Europäisches Patentamt

European Patent Office

Office européen des brevets

(11) Numéro de publication: **0 239 504 B1**

# FASCICULE DE BREVET EUROPEEN

(45) Date de publication de fascicule du brevet:
12.06.91

(51) Int. Cl.⁵: **C12N 1/20**, C12N 1/38, A61K 39/10

(21) Numéro de dépôt: 87400697.6

(22) Date de dépôt: 27.03.87

(54) **Nouveaux milieux de culture de bactéries appartenant au genre Bordetella, contenant des dérivés éthérifiés de polymères de D-glucose, et leur application.**

(30) Priorité: 27.03.86 FR 8604440

(43) Date de publication de la demande:
30.09.87 Bulletin 87/40

(45) Mention de la délivrance du brevet:
12.06.91 Bulletin 91/24

(84) Etats contractants désignés:
AT BE CH DE ES GB GR IT LI LU NL SE

(56) Documents cités:
EP-A- 0 077 646
EP-A- 0 121 249
US-A- 4 551 429

(73) Titulaire: **PASTEUR-MERIEUX Sérums et Vaccins**
**58 avenue Leclerc**
**F-69007 Lyon(FR)**

(72) Inventeur: **Quentin-Millet, Marie-José B.J.**
**70, Cours Emile Zola**
**F-69100 Villeurbanne(FR)**
Inventeur: **Arminjon, François**
**17, Boulevard de la Croix-Rousse**
**F-69004 Lyon(FR)**
Inventeur: **Donikian, Roupen Robert**
**124, rue Sully**
**F-69006 Lyon(FR)**

(74) Mandataire: **Nony, Michel et al**
**Cabinet NONY & CIE, 29, rue Cambacérès**
**F-75008 Paris(FR)**

## Description

La présente invention a pour objet une nouvelle composition de milieu de culture de bactéries appartenant au genre Bordetella, ce milieu de culture contenant au moins un dérivé éthérifié de polymère de D-glucose. L'invention concerne également une méthode de culture desdites bactéries à l'aide d'un tel milieu.

On sait que la culture de bactéries du genre Bordetella, par exemple Bordetella pertussis, Bordetella parapertussis et Bordetella bronchiseptica, soulève des problèmes difficiles, notamment dans la mise au point de milieux de culture purement synthétiques.

La bactérie Bordetella pertussis est l'agent pathogène de la coqueluche, et sa culture industrielle est nécessaire pour produire des vaccins anticoquelucheux acellulaires ou à germes entiers.

On sait également que la culture de Bordetella pertussis, phase I, permet l'obtention, dans le milieu de culture, de la toxine pertussis (encore appelée LPF ou LPF-HA : Leukocytosis Promoting Factor-Hemagglutinin) et le F-HA (Filamentous Hemagglutinin) qui sont des substances antigéniques susceptibles d'entrer dans la composition d'un vaccin acellulaire anticoquelucheux.

Les bactéries du genre Bordetella ont été d'abord cultivées sur un milieu solide appelé milieu de Bordet-Gengou.

On a proposé depuis diverses compositions de milieux liquides pour la culture des bactéries appartenant au genre Bordetella, en particulier Bordetella pertussis en phase I.

Le premier milieu liquide pour la culture des bactéries Bordetella pertussis en phase I a été décrit par Hornibrook J.W., Public Health Reports, 54 , n° 41, 1847-1851 (1939). Le milieu décrit par Hornibrook contient de l'amidon. Hornibrook a signalé en outre qu'il est possible de remplacer l'amidon par la bêta-dextrine ou par l'alpha-dextrine. Ces dextrines, maintenant appelées alpha et bêta-cyclodextrine, sont des polyosides obtenus par l'action de B. macerans sur l'amidon de pomme de terre.

Plusieurs auteurs ont ensuite apporté des modifications et améliorations au milieu décrit par Hornibrook et un milieu liquide purement synthétique a été mis au point par Stainer et Scholte, J. Gen. Microbiol., 63, 211-220 (1970).

Le milieu de culture de Stainer et Scholte, appelé couramment milieu SS, est chimiquement défini. Il est dépourvu de substances naturelles ou de leurs dérivés, tels que des extraits de levures, des polypeptones, etc..

L'absence de telles substances complexes dans le milieu rend plus aisée la purification des antigènes protéiques excrétés par les microorganismes, comme la toxine pertussis et le F-HA. Le milieu SS permet en outre une bonne reproductibilité de la qualité des suspensions de germes obtenues en fin de culture.

Le milieu SS contient essentiellement, en proportions définies, les produits suivants : Glutamate de sodium, L-proline, chlorure de sodium, dihydrogénophosphate de potassium, chlorure de potassium, chlorure de magnésium, chlorure de calcium, et tris-hydroxyméthylaminométhane, auxquels on ajoute les produits suivants : L-cystine, sulfate ferreux, acide ascorbique, glutathion réduit et niacine.

Dans un milieu SS modifié, on ajoute en outre une solution de casamino-acides ou de peptides correspondants.

Toutefois, le milieu SS ne permet pas l'expression des antigènes déjà cités en quantité suffisante compatible avec un développement industriel. De plus, avec ce milieu liquide, l'antigène F-HA ne peut être produit que lorsque la culture est réalisée dans des conditions statiques.

Pour produire la toxine pertussis et le F-HA dans un milieu liquide agité, Imaizumi et al., J. Clin. Microbiol., 17, n°5, 781-786 (1983), ont défini un milieu de Stainer et Scholte modifié dans lequel ils ont ajouté un dérivé méthylé de la bêta-cyclodextrine. Ce polyoside cyclique, encore appelé Heptakis (2,6-0-diméthyl) bêta-cyclodextrine, augmente à la fois la biomasse et la production de toxine pertussis et de F-HA.

Toutefois, l'utilisation de ce dérivé méthylé de la bêta-cyclodextrine soulève des problèmes économiques, en raison de l'étape de préparation du dérivé diméthylé en position 2,6, à partir de la bêta-cyclodextrine, par réaction enzymatique.

Un des objets de la présente invention est une composition de milieu de culture à base de composés chimiquement définis, capable de favoriser le développement des bactéries du genre Bordetella, et en particulier de favoriser la production simultanée des antigènes toxine pertussis et F-HA avec un rendement élevé. Ce nouveau milieu de culture permet d'éviter l'emploi coûteux de dérivés éthérifiés de la cyclodextrine.

On a maintenant découvert que les dérivés éthérifiés de polymères de D-glucose de masse moléculaire au moins égale à 2000 n'ont d'effet ni sur la production de la toxine pertussis, ni sur la production de F-HA, et même inhibent la croissance des cultures de bactéries du genre Bordetella, mais que, de façon surprenante, ces dérivés éthérifiés, lorsqu'ils sont associés à la cyclodextrine ou ses dérivés, favorisent le développement microbien et/ou la production simultanée de toxine pertussis et de F-HA avec un rendement élevé.

L'invention a donc pour objet une nouvelle composition de milieu de culture des bactéries appartenant au genre Bordetella, comprenant les ingrédients de base nécessaires à cette culture, ainsi qu'une cyclodextrine ou ses dérivés, caractérisée par le fait que ladite composition contient en outre au moins un dérivé éthérifié d'un polymère de D-glucose dont la masse moléculaire est au moins égale à 2000.

Les polymères de D-glucose dont dérivent les produits éthérifiés utilisés selon l'invention sont des polymères dont les motifs D-glucose sont réunis par une liaison alpha comme dans l'amylose, ou par une liaison bêta comme dans la cellulose.

Parmi les dérivés éthérifiés de polymères de D-glucose, on citera notamment les dérivés éthérifiés de la cellulose dans lesquels une partie des groupements hydroxyle sont éthérifiés par des groupements alkyle inférieur ou hydroxy-alkyle inférieur. Parmi les dérivés éthérifiés de la cellulose, on citera en particulier la méthylcellulose, l'hydroxypropyl méthylcellulose, l'hydroxyéthyl cellulose, l'hydroxybutyl méthylcellulose etc... ; tous ces produits sont des produits commerciaux vendus notamment par les firmes Dow Chemical, Colorcon, Union Carbide Corporation et Hercules Inc. On citera également la carboxyméthylcellulose qui est également un produit commercial, vendu notamment par les firmes Hercules Inc. ou E.I. du Pont de Nemours. Parmi les autres dérivés éthérifiés de polymères de D-glucose, on citera aussi les dérivés correspondants de l'amidon, et en particulier de l'amylose.

La limite supérieure du poids moléculaire des dérivés éthérifiés de polymères de D-glucose n'est pas déterminante. De préférence, on choisit le dérivé éthérifié de façon que le poids moléculaire et le degré de substitution soient tels que le dérivé aux concentrations utilisées soit soluble dans l'eau. Généralement le milieu de culture de l'invention contient de 0,01 à 2g/litre du dérivé éthérifié du polymère de D-glucose, et de préférence de 0,05 à 0,5g/litre.

Le dérivé éthérifié peut être ajouté dans le milieu en plusieurs fois, généralement de 2 à 6 fois ; on ajoute par exemple à chaque fois de 0,01 à 1g/litre dudit dérivé.

La cyclodextrine (ou un de ses dérivés) est présente dans le milieu de culture de l'invention à raison de 0,5 à 5g/litre, de préférence de 1 à 2g/litre.

La cyclodextrine peut être l'alpha-cyclodextrine, la bêta-cyclodextrine ou la gamma-cyclodextrine. Les dérivés de cyclodextrine sont notamment les dérivés éthérifiés.

L'invention a également pour objet l'utilisation des dérivés éthérifiés de polymères de D-glucose, tels que définis ci-dessus, en association avec la cyclodextrine ou ses dérivés, dans la culture de bactéries du genre Bordetella, et en particulier dans la culture en milieu agité de Bordetella pertussis, pour favoriser la croissance de la culture et/ou l'expression des antigènes toxine pertussis et F-HA.

L'invention a en particulier pour objet un procédé de culture des bactéries du genre Bordetella, à l'aide de la composition de milieu de culture décrite ci-dessus. Ce procédé, qui consiste à cultiver lesdites bactéries dans un milieu de culture approprié, le milieu de culture contenant une cyclodextrine ou ses dérivés, est caractérisé par le fait que l'on ajoute audit milieu de culture au moins un dérivé éthérifié de polymère de D-glucose, tel que défini ci-dessus.

Ledit dérivé éthérifié de polymère de D-glucose peut être ajouté en une seule fois en début de culture, ou en plusieurs fois, comme indiqué précédemment, de façon à utiliser de 0,05 à 2g/litre, et de préférence de 0,05 à 0,5g/litre, dudit dérivé.

On décrit ci-après un mode d'exécution particulier du procédé de l'invention, en milieu liquide agité.

Parmi les milieux de culture de base utilisables dans le procédé de l'invention, on citera notamment un milieu SS modifié ayant la composition suivante (pour 1 litre) :

| | |
|---|---|
| – glutamate de sodium | 10,72 g |
| – L-proline | 0,24 g |
| – $KH_2PO_4$ | 0,50 g |
| – KCl | 0,20 g |
| – $MgCl_2,6H_2O$ | 0,10 g |
| – $CaCl_2$ | 0,02 g |
| – Tris | 6,10 g |
| – Casamino acides* | 10,00 g |
| – NaCl | 2,50 g |
| – L-cystine | 40,00mg |
| – $FeSO_4, 7H_2O$ | 10,00mg |
| – Acide ascorbique | 20,00mg |
| – Glutathion réduit | 100,00mg |
| – Niacine | 4,00mg |

* ou peptides correspondants préparés par hydrolyse enzymatique de la caséine.

On effectue les précultures dans le milieu SS modifié contenant ou non une cyclodextrine.

La culture proprement dite est effectuée par ensemencement d'un fermenteur avec une concentration en germes de l'ordre de $10^8$ ou $10^9$ germs/ml. La concentration en germes est évaluée par la lecture de la densité optique en analyse spectrophotométrique à 650nm.

Le milieu de culture dans le fermenteur est le milieu SS modifié contenant une cyclodextrine à une concentration de 1,7g/litre.

La culture est effectuée à 35-37°C, de préférence à 36°C.

On opère en milieu agité avec aération.

Le dérivé éthérifié de polymère de D-glucose utilisé est ici une celullose ayant un poids moléculaire moyen de 9.300, et dont certains groupements alcool ont été méthylés (% de substitution : environ 30% en poids).

Lorsque l'addition de méthylcellulose est réalisée en une seule fois, la quantité ajoutée est de préférence 0,1g/litre environ. Cette addition peut se faire lorsque le germe est en phase de croissance exponentielle, généralement après 24heures de culture.

Lorsque l'addition est réalisée en plusieur fois, la quantité totale ajoutée est également, de préférence, 0,1g/litre.

On peut ajouter la solution de méthylcellulose en 4 fois, par exemple après 7 heures, 24 heures, 33 heures et 38 heures de culture.

L'evolution de la biomasse est appréciée par la mesure au cours du temps de la densité optique à 650nm et par mesure de l'opacité à l'aide d'un standard OMS équivalent à 10 "opacity Units" par ml, une unité correspondant à un milliard de germes/ml.

La teneur en antigènes toxine pertussis et F-HA dans le surnageant est estimée par un test ELISA à l'aide d'anticorps anti-F-HA et d'anticorps anti-toxine pertussis purifiés par immunoaffinité à partir de sera hyperimmunisés respectivement de chèvre et d'âne.

La durée de culture dépend du développement microbien et de l'expression dans le surnageant de culture de la toxine pertussis et du F-HA. Elle sa situe entre 30 et 72 heures, et le plus souvent entre 40 et 45 heures.

Les exemples suivants illustrent l'invention sans toutefois la limiter.

## EXEMPLE 1

Afin de mettre en évidence l'effet favorable de la composition de milieu de l'invention sur le développement de B. pertussis en phase I et sur la production dans le milieu de culture des antigènes toxine pertussis et F-HA, on a réalisé une étude comparative des formules de milieu suivantes :
- milieu de Stainer et Scholte modifié (apport d'hydrolysat de caséine) ;

- milieu A = milieu de Stainer et Scholte modifié contenant la bêta-cyclodextrine à une concentration de 1,67g/litre ;
- milieu de Stainer et Scholte modifié contenant de la bêta-2,6-di-O-méthylcyclodextrine à la concentration finale de 1,67g/litre (milieu défini par Imaizumi A., Suzuki Y., Ono S., Sato H. and Sato Y. (1983) J. Clin. Microbiol., 17, n° 5, 781-786) ;
- milieu B = identique au milieu A, avec en outre additions ponctuelles de méthylcellulose type A15 commercialisée par la société Colorcon, comme décrit ci-dessus.

Ces milieux ont été essayés en fermenteur de capacité utile 30 litres avec B. pertussis en phase I souche TOHAMA.

Ces cultures ont été réalisées dans des conditions de fermentation identiques (agitation, aération, pH, durée, etc...)

On a évalué à intervalles réguliers la biomasse et la teneur en toxine pertussis et F-HA dans le surnageant.

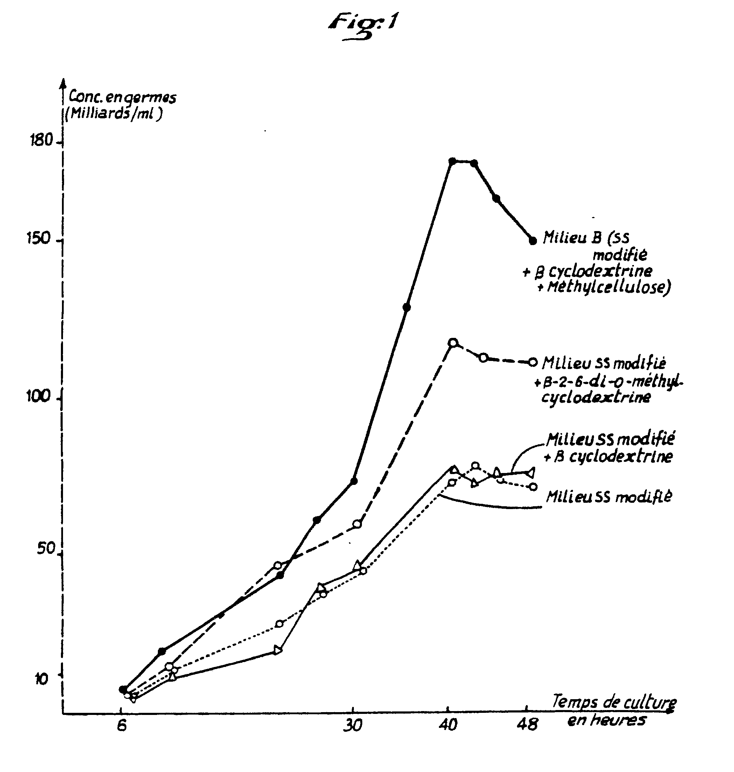

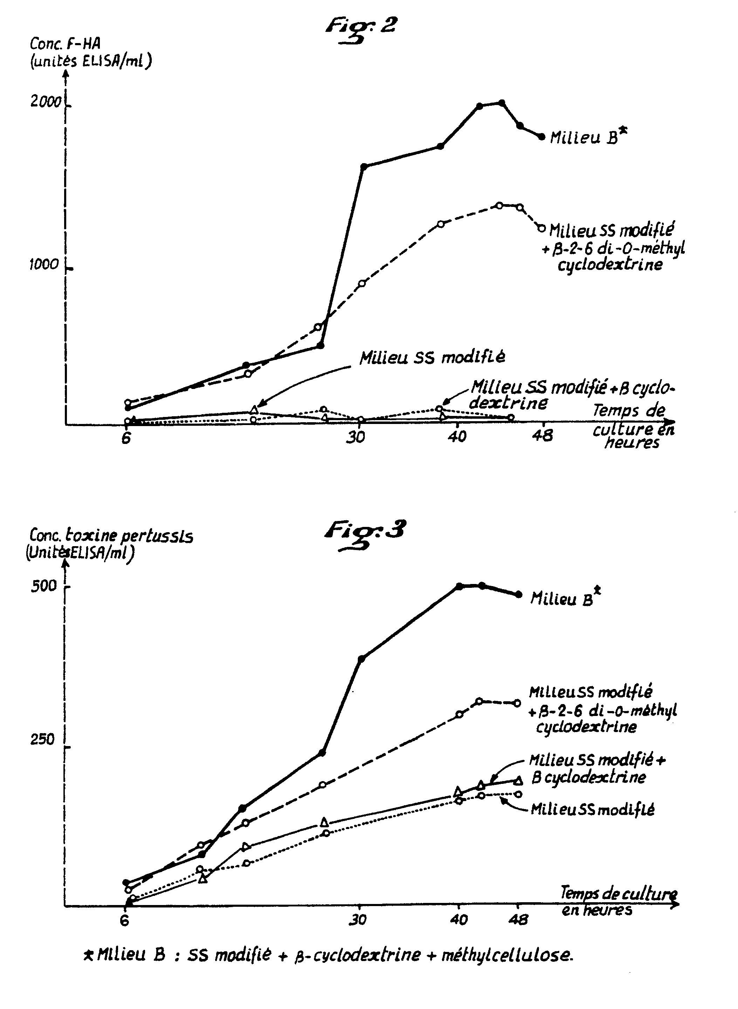

Les résultats sont indiqués dans les figures 1, 2 et 3 ci-après.

La figure 1 représente l'évolution dans le temps de la biomasse en fonction de la composition du milieu de culture ;

La figure 2 représente l'évolution dans le temps de la production de F-HA en fonction de la composition du milieu de culture ;

La figure 3 représente l'évolution dans le temps de la production de toxine pertussis en fonction de la composition du milieu de culture.

A l'analyse des courbes obtenues il apparaît que :

L'addition seule de bêta-cyclodextrine au milieu de Stainer et Scholte ne permet pas d'augmenter la biomasse, ni la production des antigènes toxine pertussis et F-HA. Dans le milieu et dans les conditions de fermentation utilisées (avec agitation), il n'y a pas de production de F-HA dans le milieu de culture.

Par contre, la substitution de la bêta-cyclodextrine par son dérivé 2,6- di-O-méthylé favorise le développement microbien et la production de toxine pertussis et F-HA.

Enfin, le milieu B (avec addition de méthylcellulose), augmente encore davantage la biomasse et la production de toxine pertussis et de F-HA dans le milieu.

La souche TOHAMA est une souche déposée auprès de la collection Institute for Fermentation, Osaka (Japon) sous le n° IFO-14073).

Elle est en outre disponible auprès de la Japanese Federation of Culture Collections of Microorganisms, Institute of Medical Science, University of Tokyo, Shirojanedai-461, Milato-ku, Tokyo 108 (Japon).


EXEMPLE 2

Afin d'évaluer l'influence de différents dérivés de la cellulose sur le développement de la biomasse et sur l'expression des antigènes toxine pertussis et F-HA dans le surnageant de culture, une série d'essais de culture en erlens ont été réalisés.

A l'aide de deux ampoules de B. pertussis souche TOHAMA en phase I lyophilisées, on a ensemencé 8 tubes contenant le milieu solide de Bordet-Gengou. Après incubation pendant 68 heures à 36° C, en atmosphère humide, chaque tube est repris par environ 25ml de milieu A tel que défini à l'exemple 1 (SS modifié avec bêta-cyclodextrine). La suspension de germes obtenue est utilisée pour ensemencer 4 erlens de 2 litres contenant 500ml de milieu A. Les erlens sont mis en incubation pendant 24 heures à 36° C sous agitation.

Les suspensions bactériennes sont réunies et le mélange sert à ensemencer 18 erlens de 2 litres renfermant 450ml de milieu A. Ils sont mis en incubation sous agitation pendant 48 heures. Au bout de 24 heures, chaque série de 2 erlens reçoit 25ml d'une solution contenant 1,25g/litre d'un des dérivés de la cellulose indiqués dans le tableau I ci-après.

Après 42 heures de culture, la concentration en toxine pertussis et F-HA dans chaque milieu testé est déterminée par méthode immunoenzymatique. Les résultats sont regroupés dans le tableau I.

TABLEAU I

| Dérivé de cellulose ajouté au milieu A | Tox. pertussis unités ELISA/ml | F-HA unités ELISA/ml |
|---|---|---|
| Témoins : milieu A seul | 12 | 50 |
| Methocel A15 | 50 | 1560 |
| Methocel A4C | 25 | 825 |
| Methocel A4M | 25 | 325 |
| Methocel E5 | ND | 1100 |
| Methocel E15 | 15 | 1562 |
| Methocel E4M | · 27 | 1100 |
| Carboxyméthylcellulose type Akucel AF 2805 | 17 | 50 |
| AF 2205 | 20 | 50 |

Les dérivés méthylés de la cellulose type méthocel A15, A4C et A4M commercialisés par la Société Colorcon, ont une influence très favorable sur la production des antigènes toxine pertussis et F-HA. La teneur en toxine pertussis dans le milieu de culture, après 42 heures de culture, est 2 à 4 fois plus élevée que celle obtenue avec le milieu A et, en ce qui concerne la concentration en F-HA, 6 à 30 fois plus forte. Dans les conditions utilisées, les meilleurs rendements ont été obtenus avec la méthylcellulose type A15.

Les dérivés carboxyméthylés ont un effet favorable sur l'expression de la toxine pertussis. La production de toxine pertussis est cependant moins élevée que celle obtenue à l'aide des dérivés méthylés de la cellulose. Par contre, dans les conditions étudiées, les dérivés carboxyméthylés de la cellulose n'ont aucune influence sur la production de F-HA.

EXEMPLE 3

Cet exemple décrit le procédé de fermentation de la souche TOHAMA de Bordetella pertussis en phase I dans le milieu B (SS modifié + bêta-cyclodextrine + méthylcellulose), en fermenteur de volume utile de 1000 litres. Le but de cette culture est de produire dans le surnageant de culture des quantités importantes d'antigènes toxine pertussis et F-HA, compatibles avec la réalisation à l'échelle industrielle d'un vaccin anti-coquelucheux acellulaire composé des principes actifs hautement purifiés : anatoxine pertussis et/ou F-HA.

Cette culture industrielle comprend les étapes suivantes :

PRECULTURE 1

Des tubes contenant le milieu solide de Bordet Gengou sont ensemencés avec la souche TOHAMA de Bordetella pertussis en phase I.

Cinq ampoules lyophilisées, reprises chacune par 1 ml de milieu trypticase soja sont utilisées pour ensemencer 30 tubes renfermant le milieu de Bordet-Gengou. Les tubes sont placés à l'étuve à 36° C sous atmosphère humide pendant 60 heures.

PRECULTURE 2

Chaque tube est repris par environ 25ml de milieu A. Après contrôle de la pureté par l'essai de Gram, les suspensions de germes récupérées sont mélangées. Le mélange est utilisé pour ensemencer 16 erlens de 2 litres contenant chacun 400ml de milieu A. Chacun erlen reçoit environ 50ml du mélange de la suspension de germes. Les erlens sont mis en incubation à 36° C pendant 24 heures sous agitation type va et vient.

Après examen de la pureté des erlens par l'essai de Gram et le contrôle de l'absence de développement de contaminants sur les milieux de Bordet-Gengou, les suspensions bactériennes des erlens sont réunies et la concentration en germes est estimée par mesure de la densité optique à 650nm. Préculture 2 : $DO_{650}nm = 1,80$ correspondant à environ $40.10^9$ germes/ml.

PRECULTURE 3 : Stade préfermenteurs

Deux fermenteurs renfermant 40 litres de milieu A sont ensemencés chacun avec 3,2 litres de la préculture précédente, pour avoir une suspension de germes titrant au moins $10^9$ germes/ml.

La culture est réalisée à 36° C, sous agitation modérée et sous aération pendant 24 heures.

En fin de fermentation, la pureté est contrôlée par essai de Gram et la densité de germes appréciée par la mesure de la densité optique à 650nm. Après 24 heures d'incubation les densités optiques mesurées sur chaque préfermenteur étaient égales à 3,01 et 3,37, ce qui correspond à une concentration bactérienne d'environ $64-68.10^9$ germes/ml.

CULTURE INDUSTRIELLE

La culture industrielle a été réalisée en fermenteur contenant 1000 litres de milieu B tel que défini à l'exemple 1. Ce fermenteur a été ensemencé avec la préculture précédente.

La concentration en germes après ensemencement est de l'ordre de $5.10^9$ germes/ml.

La culture est effectuée à 36° C, sous agitation pendant 40 heures, avec aération.

En cours de fermentation, on ajoute en plusieurs fois une solution de méthylcellulose type Colorcon A15, en fonction de l'évolution de la biomasse au cours du temps. Les additions de méthylcellulose ont été effectuées au bout de 7h, 24h et 32h de culture. Chaque addition consistait à transférer dans le fermenteur 24 litres d'une solution de milieu A contenant 1,25g/litre de méthylcellulose.

La culture est arrêtée à t = 40 heures.

Après refroidissement, la culture est inactivée par l'addition de merthiolate à la concentration finale de 0,01% (P/V), et centrifugée en continu.

Le surnageant est clarifié par filtration.

L'évolution de la biomasse ainsi que la teneur en antigènes (mesurée par un test ELISA) dans la surnageant de culture, au cours du temps, sont résumées dans le tableau II ci-après.

EP 0 239 504 B1

## TABLEAU II

| Temps de culture | $DO_{650}$ | Concentration en germes par ml | Teneur en F-HA Unités ELISA par ml | Teneur en toxine pertussis Unités ELISA par ml |
|---|---|---|---|---|
| t 0 | 0,301 | $7,5.10^9$ | - | - |
| t 6h | 0,77 | $15.10^9$ | - | - |
| t 22h | 3,54 | $72.10^9$ | - | - |
| t 24h | 4,10 | $90.10^9$ | 450 | 240 |
| t 30h | 5,97 | $120.10^9$ | 1800 | 480 |
| t 40h | 8,20 | $170.10^9$ | 2200 | 550 |

## Revendications

1. Composition de milieu de culture des bactéries appartenant au genre Bordetella, comprenant les ingrédients de base nécessaires à cette culture, ainsi que la cyclodextrine ou ses dérivés, caractérisée par le fait que ladite composition contient en outre au moins un dérivé éthérifié d'un polymère de D-glucose ayant une masse moléculaire au moins égale à 2000.

2. Composition selon la revendication 1, caractérisée par le fait que ledit dérivé éthérifié est un dérivé de la cellulose dans lequel une partie des groupements hydroxyle sont éthérifiés par des groupements alkyle inférieur ou hydroxy-alkyle inférieur.

3. Composition selon la revendication 2, caractérisée par le fait que lesdits dérivés éthérifiés de la cellulose sont choisis parmi la méthylcellulose, l'hydroxypropyl méthylcellulose, l'hydroxyéthyl cellulose, l'hydroxybutyl méthylcellulose.

4. Composition selon la revendication 1, caractérisée par le fait que ledit dérivé éthérifié est la carboxyméthyl cellulose.

5. Composition selon l'une quelconque des revendications précédentes, caractérisée par le fait que le milieu de culture contient de 0,01 à 2g/litre dudit dérivé éthérifié, et de préférence de 0,05 à 0,5g/litre.

6. Composition selon l'une quelconque des revendications précédentes, caractérisée par le fait que la cyclodextrine, ou un de ses dérivés, est présente à raison de 0,5 à 5g/litre, de préférence de 1 à 2g/litre.

7. Utilisation d'un dérivé éthérifié d'un polymère de D-glucose ayant une masse moléculaire au moins égale à 2000, en association avec la cyclodextrine ou ses dérivés, dans la culture de bactéries du genre Bordetella, et en particulier dans la culture en milieu agité de Bordetella pertussis, pour favoriser la croissance de la culture et/ou l'expression des antigènes LPF et F-HA.

8. Utilisation selon la revendication 7, caractérisée par le fait que ledit dérivé éthérifié est tel que défini dans l'une quelconque des revendications 2 à 4.

8

9. Procédé de culture des bactéries du genre Bordetella consistant à cultiver lesdites bactéries dans un milieu de culture approprié, le milieu de culture contenant une cyclodextrine ou ses dérivés, caractérisé par le fait que l'on ajoute audit milieu de culture au moins un dérivé éthérifié d'un polymère de D-glucose ayant une masse moléculaire au moins égale à 2000.

10. Procédé selon la revendication 9, caractérisé par le fait que l'on ajoute ledit dérivé éthérifié, en une ou plusieurs fois, à raison de 0,05 à 2g/litre, et de préférence de 0,05 à 0,5g/litre.

11. Procédé selon la revendication 9 ou 10, caractérisé par le fait que ledit dérivé éthérifié est tel que défini dans l'une quelconque des revendications 2 à 4.

## Claims

1. Culture medium composition for bacteria belonging to the genus Bordetella, comprising the basic ingredients necessary for culturing these organisms as well as cyclodextrin or its derivatives, characterised in that the said composition contains, in addition, at least one etherified derivative of a polymer of D-glucose having a molecular mass equal to at least 2000.

2. Composition according to Claim 1, characterised in that the said etherified derivative is a cellulose derivative in which a portion of the hydroxyl groups are etherified with lower alkyl or lower hydroxyalkyl groups.

3. Composition according to Claim 2, characterised in that the said etherified derivatives of cellulose are selected from methylcellulose, hydroxypropylmethylcellulose, hydroxyethylcellulose and hydroxybutylmethylcellulose.

4. Composition according to Claim 1, characterised in that the said etherified derivative is carboxymethylcellulose.

5. Composition according to any one of the preceding claims, characterised in that the culture medium contains 0.01 to 2 g/litre of the said etherified derivative, and preferably 0.05 to 0.5 g/litre.

6. Composition according to any one of the preceding claims, characterised in that the cyclodextrin or one of its derivatives is present in the proportion of 0.5 to 5 g/litre, and preferably 1 to 2 g/litre.

7. Use of an etherified derivative of a polymer of D-glucose having a molecular mass equal to at least 2000, in combination with cyclodextrin or its derivatives, in the culture of bacteria of the genus Bordetella, and especially in the culture of Bordetella pertussis in agitated medium, to promote the growth of the culture and/or the expression of the LPF and F-HA antigens.

8. Use according to Claim 7, characterised in that the said etherified derivative is as defined in any one of Claims 2 to 4.

9. Process for culturing bacteria of the genus Bordetella, consisting in culturing the said bacteria in a suitable culture medium, the culture medium containing a cyclodextrin or its derivatives, characterised in that at least one etherified derivative of a polymer of D-glucose having a molecular mass equal to at least 2000 is added to the said culture medium.

10. Process according to Claim 9, characterised in that the said etherified derivative is added, in one or more portions, in the proportion of 0.05 to 2 g/litre, and preferably 0.05 to 0.5 g/litre.

11. Process according to Claim 9 or 10, characterised in that the said etherified derivative is as defined in any one of Claims 2 to 4.

## Ansprüche

1. Zubereitung eines Kulturmediums von Bakterien, die dem Stamm Bordetella zugehörig sind, enthaltend notwendige Grundbestandteile dieser Kultur und, und außerdem Cyclodextrin oder dessen Derivate,

9

dadurch gekennzeichnet, daß diese Zubereitung weiterhin mindestens ein veräthertes Derivat eines Polymers der D-Glucose mit einem Molekulargewicht von mindestens 2000 enthält.

2. Zubereitung nach Anspruch 1, dadurch gekennzeichnet, daß dieses veräthertes Derivat ein Cellulosederivat ist, bei dem ein Teil der Hydroxylgruppen durch niedere Alkylgruppen oder niedere Hydroxylalkylgruppen veräthert sind.

3. Zubereitung nach Anspruch 2, dadurch gekennzeichnet, daß die verätherten Derivate der Cellulose ausgewählt sind aus Methylcellulose, Hydroxypropylmethylcellulose, Hydroxyethylcellulose, Hydroxybutylmethylcellulose.

4. Zubereitung nach Anspruch 1, dadurch gekennzeichnet, daß das verätherte Derivat Carboxymethylcellulose ist.

5. Zubereitung nach den vorhergehenden Ansprüchen, dadurch gekennzeichnet, daß das Kulturmedium 0,01 bis 2 g/Liter, vorzugsweise 0,05 bis 0,5 g/Liter des verätherten Derivats enthält.

6. Zubereitung nach den vorhergehenden Ansprüchen, dadurch gekennzeichnet, daß das Cyclodextrin oder dessen Derivate in einer Menge von 0,5 bis 5 g/Liter, vorzugsweise 1 bis 2 g/Liter, vorhanden sind.

7. Verwendung eines verätherten Derivats eines Polymers der D-Glucose mit einem Molekulargewicht von mindestens 2000 in Verbindung mit dem Cyclodextrin oder einem seiner Derivate in der Kultur von Bakterien des Stammes Bordetella und insbesondere in einem gerührten Kulturmedium von Bordetella pertussis zur Begünstigung des Wachstums der Kultur und/oder der Ausbildung von Antigenen LPF und F-HA.

8. Verwendung nach Anspruch 7, dadurch gekennzeichnet, daß das verätherte Derivat ein solches ist, wie es in einem der Ansprüche 2 bis 4 definiert ist.

9. Verfahren zum Kultivieren von Bakterien des Stammes Bordetella, bestehend im Kultivieren dieser Bakterien in einem geeigneten Kulturmedium, wobei das Kulturmedium ein Cyclodextrin oder seine Derivate enthält, dadurch gekennzeichnet, daß man zu dem Kulturmedium mindestens ein veräthertes Derivat eines Polymeren der D-Glucose mit einem Molekulargewicht von mindestens 2000 zufügt.

10. Verfahren nach Anspruch 9, dadurch gekennzeichnet, daß man das verätherte Derivat auf einmal oder in mehreren Teilmengen in einer Menge von 0,05 bis 2 g/Liter, vorzugsweise 0,05 bis 0,5 g/Liter zugibt.

11. Verfahren nach Anspruch 9 oder 10, dadurch gekennzeichnet, daß das verätherte Derivat ein solches ist, wie es in den Ansprüchen 2 bis 4 definiert ist.

Fig. 1

*Fig. 2*

Conc. F-HA
(unités ELISA/ml)

2000

1000

Milieu B*

Milieu SS modifié
+ β-2-6 di-O-méthyl
cyclodextrine

Milieu SS modifié

Milieu SS modifié + β cyclodextrine

Temps de
culture en
heures

6    30    40    48

*Fig. 3*

Conc. toxine pertussis
(Unités ELISA/ml)

500

250

Milieu B*

Milieu SS modifié
+ β-2-6 di-O-méthyl
cyclodextrine

Milieu SS modifié +
β cyclodextrine

Milieu SS modifié

Temps de culture
en heures

6    30    40    48

*Milieu B : SS modifié + β-cyclodextrine + méthylcellulose.